# EUROPEAN PATENT SPECIFICATION

(11) **EP 0 355 899 B1**
(45) Date of publication and mention of the grant of the patent: **02.11.1994**
(21) Application number: 89202041.3
(22) Date of filing: 04.08.1989
(51) Int. Cl.: C07H 19/20, A61K 31/70

(54) **Nucleotide derivatives**
Nucleotid-Derivate
Dérivés de nucléotides

(30) Priority: 16.08.1988 JP 203452/88
(43) Date of publication of application: 28.02.1990
(73) Proprietor: NIPPON SHINYAKU COMPANY, LIMITED, Minami-ku Kyoto-shi Kyoto 601 (JP)
(72) Inventor: Yano, Junichi, Nara-shi 630 (JP); Ohgi, Tadaaki, Otsu-shi 520 (JP); Ishiyama, Koichi 51-22-Z-405 Koaza Katsuhara, Otokuni-gu Kyoto-fu 618 (JP); Hirabayashi, Kazuko Room 207, Green Palace, Uzumasa Ukyo-ku Kyoto 616 (JP)
(74) Representative: Kupecz, Arpad

(56) References cited:
- EP-A- 0 044 527
- EP-A- 0 061 001
- EP-A- 0 354 246
- US-A- 4 401 808
- US-A- 4 567 254
- CHEMICAL ABSTRACTS, vol. 109, no. 25, 19th December 1988, pages 909-910,abstract no. 231470p, Columbus, Ohio, US; &JP-A-63 135 399 (A. KAJI) 07-06-1988
- J.Chem. Med. vol.16, p.1319 (1973)

## Description

The present invention relates to cyclic AMP derivatives and cyclic GMP derivatives having useful pharmacological activities, such as antidementia activity,. The compounds according to the invention are novel and can be represented by the following General Formula [I]: 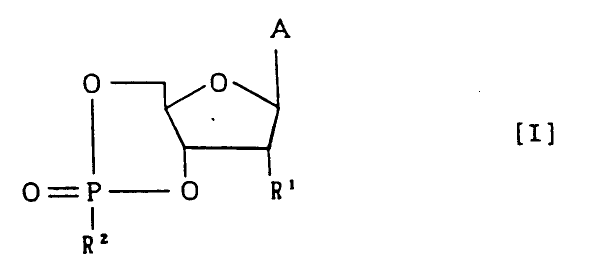
including optically pure compounds thereof
[wherein R¹ represents a hydrogen atom, a hydroxyl group or an acyloxy group; R² represents an alkyl group; and A represents: 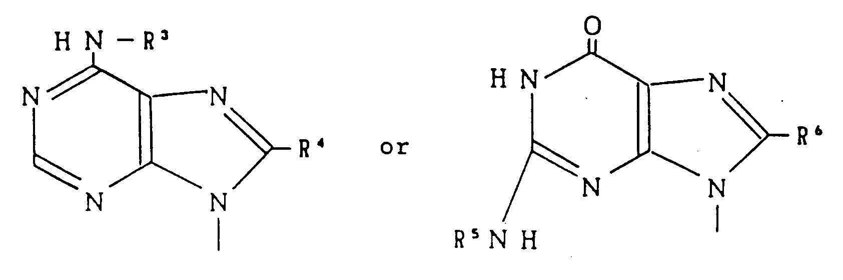
(in which R³ represents a hydrogen atom or an acyl group; R⁴ represents a hydrogen atom or a halogen atom; R⁵ represents a hydrogen atom or an acyl group; and R⁶ represents a hydrogen atom or a halogen atom)], excluding the one in which R¹ is hydroxyl, R² is alkyl and A is: 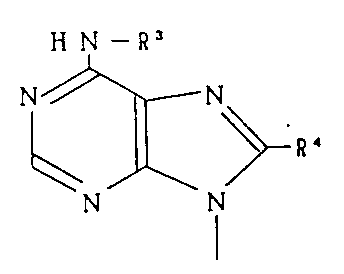
(in which R³ is hydrogen and R⁴ is hydrogen).

It has been known that cyclic AMP acts, e.g., as a transmitter of hormones and participates in various physiological reactions in living bodies. Accordingly, attention has been paid on its pharmacological effects.

There are, however, many questions on its usefulness as a medicament. For example, the compound is absorbed only poorly in living bodies and, even after being absorbed, tends to be easily decomposed by enzymes contained therein.

Dibutyryl cyclic AMP has been synthesized for the purpose of improving the cell permeability of cyclic AMP and has been in actual use as a medicament.

Cyclic AMP derivatives with modified purine moieties were synthesized and exhibited the inhibition activity of phosphodiesterase (J. Med. Chem., 16, 1319 (1973)).

A recent study proved that a compound (adenosine-3',5'-cyclic methylphosphonate), which corresponds to General Formula [I] in which R¹ is hydroxyl, R² is methyl, and A is: 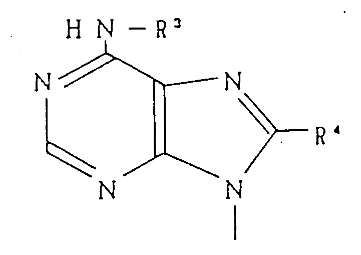
(in which R³ is hydrogen and R⁴ is hydrogen), possesses useful pharmacological activities (see Japanese Unexamined Patent Publication No. 135,399/88).

The above study is useful in that it has proved that a cyclic AMP derivative possesses pharmacological activities. However, the study disclosed only one cyclic AMP derivative in which a methyl group is contained as the only substitutional group for the phosphoric acid moiety, and nothing is disclosed with regard to other cyclic AMP derivatives.

### [Problem to be Solved by the Invention]

An object of the present invention is to provide various cyclic AMP derivatives which are useful as medicaments. Another object of the invention is to provide cyclic GMP derivatives which exhibit pharmacological activities similar to those of cyclic AMP derivatives.

The inventors have conducted studies on the synthesis of various cyclic AMP and GMP derivatives and on their pharmacological effects, and now established a process of synthesizing cyclic AMP and GMP derivatives, confirmed their pharmacological activities and completed the present invention.

Compounds according to the present invention are represented by General Formula [I] described hereinabove.

Needless to say, the compounds of the present invention include optically pure compounds of the general formula (I).

In General Formula [I], the basic skeleton of adenine or guanine is contained in A;
R³ represents a hydrogen atom or an acyl group: As examples of acyl groups, mention may be made of those represented by -COR³¹ (in which R³¹ represents an alkyl group, such as methyl, ethyl, propyl and butyl; an aryl group, such as phenyl and naphthyl; or the like);
R⁴ represents a hydrogen atom or a halogen atom: As examples of halogen atoms, mention may be made of bromine, chlorine, fluorine, etc;
R⁵ represents a hydrogen atom or an acyl group: As examples of acyl groups, mention may be made of those represented by -COR⁵¹ (in which R⁵¹ represents an alkyl group, such as methyl, ethyl, propyl and butyl; an aryl group, such as phenyl and naphthyl; or the like);
R⁶ represents a hydrogen atom or a halogen atom: As examples of halogen atoms, mention may be made of bromine, chlorine, fluorine, etc.;
R¹ represents a hydrogen atom, a hydroxyl group or an acyloxy group; and
R² represents an ordinary alkyl group, including lower alkyl groups, such as methyl, ethyl, propyl and butyl.

As examples of compounds according to the present invention, mention may be those set forth below. However, the invention is not limited to these.

Needless to say, the present invention covers both diastereomeric and optically pure forms of those compounds.
N-acetyladenosine-3',5'-cyclic methylphosphonate
N-butyryladenosine-3',5'-cyclic methylphosphonate
N-benzoyladenosine-3',5'-cyclic methylphosphonate
N-acetyladenosine-3',5'-cyclic ethylphosphonate
N-butyryladenosine-3',5'-cyclic ethylphosphonate
N-benzoyladenosine-3',5'-cyclic ethylphosphonate
N-acetyladenosine-3',5'-cyclic propylphosphonate
N-butyryladenosine-3',5'-cyclic propylphosphonate
N-benzoyladenosine-3',5'-cyclic propylphosphonate
2'-deoxyadenosine-3',5'-cyclic methylphosphonate
N-acetyl-2'-deoxyadenosine-3',5'-cyclic methylphosphonate
N-butyryl-2'-deoxyadenosine-3',5'-cyclic methylphosphonate
N-benzoyl-2'-deoxyadenosine-3',5'-cyclic methylphosphonate
N-acetyl-2'-deoxyadenosine-3',5'-cyclic ethylphosphonate
N-butyryl-2'-deoxyadenosine-3',5'-cyclic ethylphosphonate
N-benzoyl-2'-deoxyadenosine-3',5'-cyclic ethylphosphonate
N-acetyl-2'-deoxyadenosine-3',5'-cyclic propylphosphonate
N-butyryl-2'-deoxyadenosine-3',5'-cyclic propylphosphonate
N-benzoyl-2'-deoxyadenosine-3',5'-cyclic propylphosphonate
2'-O-butyryladenosine-3',5'-cyclic methylphosphonate
N-acetyl-2'-O-butyryladenosine-3',5'-cyclic methylphosphonate
N-butyryl-2'-O-butyryladenosine-3',5'-cyclic methylphosphonate
N-benzoyl-2'-O-butyryladenosine-3',5'-cyclic methylphosphonate
N-acetyl-2'-O-butyryladenosine-3',5'-cyclic ethylphosphonate
N-butyryl-2'-O-butyryladenosine-3'5'-cyclic ethylphosphonate
N-benzoyl-2'-O-butyryladenosine-3',5'-cyclic ethylphosphonate
N-acetyl-2'-O-butyryladenosine-3',5'-cyclic propylphosphonate
N-butyryl-2'-O-butyryladenosine-3',5'-cyclic propylphosphonate
N-benzoyl-2'-O-butyryladenosine-3',5'-cyclic propylphosphonate
N-acetyl-8-bromoadenosine-3',5'-cyclic methylphosphonate
N-butyryl-8-bromoadenosine-3',5'-cyclic methylphosphonate
N-benzoyl-8-bromoadenosine-3',5'-cyclic methylphosphonate
N-acetyl-8-bromoadenosine-3',5'-cyclic ethylphosphonate
N-butyryl-8-bromoadenosine-3',5'-cyclic ethylphosphonate
N-benzoyl-8-bromoadenosine-3',5'-cyclic ethylphosphonate
N-acetyl-8-bromoadenosine-3',5'-cyclic propylphosphonate
N-butyryl-8-bromoadenosine-3',5'-cyclic propylphosphonate
N-benzoyl-8-bromoadenosine-3',5'-cyclic propylphosphonate
N-acetyl-8-bromo-2'-deoxyadenosine-3',5'-cyclic methylphosphonate
N-butyryl-8-bromo-2'-deoxyadenosine-3',5'-cyclic methylphosphonate
N-benzoyl-8-bromo-2'-deoxyadenosine-3',5'-cyclic methylphosphonate
N-acetyl-8-bromo-2'-deoxyadenosine-3',5'-cyclic ethylphosphonate
N-butyryl-8-bromo-2'-deoxyadenosine-3',5'-cyclic ethylphosphonate
N-benzoyl-8-bromo-2'-deoxyadenosine-3',5'-cyclic ethylphosphonate
N-acetyl-8-bromo-2'-deoxyadenosine-3',5'-cyclic propylphosphonate
N-butyryl-8-bromo-2'-deoxyadenosine-3',5'-cyclic propylphosphonate
N-benzoyl-8-bromo-2'-deoxyadenosine-3',5'-cyclic propylphosphonate
N-acetyl-8-bromo-2'-O-butyryladenosine-3',5'-cyclic methylphosphonate
N-butyryl-8-bromo-2'-O-butyryladenosine-3',5'-cyclic methylphosphonate
N-benzoyl-8-bromo-2'-O-butyryladenosine-3',5'-cyclic methylphosphonate
N-acetyl-8-bromo-2'-O-butyryladenosine-3',5'-cyclic ethylphosphonate
N-butyryl-8-bromo-2'-O-butyryladenosine-3',5'-cyclic ethylphosphonate
N-benzoyl-8-bromo-2'-O-butyryladenosine-3',5'-cyclic ethylphosphonate
N-acetyl-8-bromo-2'-O-butyryladenosine-3',5'-cyclic propylphosphonate
N-butyryl-8-bromo-2'-O-butyryladenosine-3',5'-cyclic propylphosphonate
N-benzoyl-8-bromo-2'-O-butyryladenosine-3',5'-cyclic propylphosphonate
Guanosine-3',5'-cyclic methylphosphonate
N-acetylguanosine-3',5'-cyclic methylphosphonate
N-butyrylguanosine-3',5'-cyclic methylphosphonate
N-benzoylguanosine-3',5'-cyclic methylphosphonate
N-acetylguanosine-3',5'-cyclic ethylphosphonate
N-butyrylguanosine-3',5'-cyclic ethylphosphonate
N-benzoylguanosine-3',5'-cyclic ethylphosphonate
N-acetylguanosine-3',5'-cyclic propylphosphonate
N-butyrylguanosine-3',5'-cyclic propylphosphonate
N-benzoylguanosine-3',5'-cyclic propylphosphonate
2'-deoxyguanosine-3',5'-cyclic methylphosphonate
N-acetyl-2'-deoxyguanosine-3',5'-cyclic methylphosphonate
N-butyryl-2'-deoxyguanosine-3',5'-cyclic methylphosphonate
N-benzoyl-2'-deoxyguanosine-3',5'-cyclic methylphosphonate
N-acetyl-2'-deoxyguanosine-3',5'-cyclic ethylphosphonate
N-butyryl-2'-deoxyguanosine-3',5'-cyclic ethylphosphonate
N-benzoyl-2'-deoxyguanosine-3',5'-cyclic ethylphosphonate
N-acetyl-2'-deoxyguanosine-3',5'-cyclic propylphosphonate
N-butyryl-2'-deoxyguanosine-3',5'-cyclic propylphosphonate
2'-O-butyrylguanosine-3',5'-cyclic methylphosphonate
N-acetyl-2'-O-butyrylguanosine-3',5'-cyclic methylphosphonate
N-butyryl-2'-O-butyrylguanosine-3',5'-cyclic methylphosphonate
N-benzoyl-2'-O-butyrylguanosine-3',5'-cyclic methylphosphonate
N-acetyl-2'-O-butyrylguanosine-3',5'-cyclic ethylphosphonate
N-butyryl-2'-O-butyrylguanosine-3',5'-cyclic ethylphosphonate
N-benzoyl-2'-O-butyrylguanosine-3'5'-cyclic ethylphosphonate
N-acetyl-2'-O-butyrylguanosine-3',5'-cyclic propylphosphonate
N-butyryl-2'-O-butyrylguanosine-3',5'-cyclic propylphosphonate
N-benzoyl-2'-O-butyrylguanosine-3',5'-cyclic propylphosphonate
N-acetyl-8-bromoguanosine-3',5'-cyclic methylphosphonate
N-butyryl-8-bromoguanosine-3',5'-cyclic methylphosphonate
N-benzoyl-8-bromoguanosine-3',5'-cyclic methylphosphonate
N-acetyl-8-bromoguanosine-3',5'-cyclic ethylphosphonate
N-butyryl-8-bromoguanosine-3',5'-cyclic ethylphosphonate
N-benzoyl-8-bromoguanosine-3',5'-cyclic ethylphosphonate
N-acetyl-8-bromoguanosine-3',5'-cyclic propylphosphonate
N-butyryl-8-bromoguanosine-3',5'-cyclic propylphosphonate
N-benzoyl-8-bromoguanosine-3',5'-cyclic propylphosphonate
N-acetyl-8-bromo-2'-deoxyguanosine-3',5'-cyclic methylphosphonate
N-butyryl-8-bromo-2'-deoxyguanosine-3',5'-cyclic methylphosphonate
N-benzoyl-8-bromo-2'-deoxyguanosine-3',5'-cyclic methylphosphonate
N-acetyl-8-bromo-2'-deoxyguanosine-3',5'-cyclic ethylphosphonate
N-butyryl-8-bromo-2'-deoxyguanosine-3',5'-cyclic ethylphosphonate
N-benzoyl-8-bromo-2'-deoxyguanosine-3',5'-cyclic ethylphosphonate
N-acetyl-8-bromo-2'-deoxyguanosine-3',5'-cyclic propylphosphonate
N-butyryl-8-bromo-2'-deoxyguanosine-3',5'-cyclic propylphosphonate
N-benzoyl-8-bromo-2'-deoxyguanosine-3',5'-cyclic propylphosphonate
N-acetyl-8-bromo-2'-O-butyrylguanosine-3',5'-cyclic methylphosphonate
N-butyryl-8-bromo-2'-O-butyrylguanosine-3',5'-cyclic methylphosphonate
N-benzoyl-8-bromo-2'-O-butyrylguanosine-3',5'-cyclic methylphosphonate
N-acetyl-8-bromo-2'-O-butyrylguanosine-3',5'-cyclic ethylphosphonate
N-butyryl-8-bromo-2'-O-butyrylguanosine-3',5'-cyclic ethylphosphonate
N-benzoyl-8-bromo-2'-O-butyrylguanosine-3',5'-cyclic ethylphosphonate
N-acetyl-8-bromo-2'-O-butyrylguanosine-3',5'-cyclic propylphosphonate
N-butyryl-8-bromo-2'-O-butyrylguanosine-3',5'-cyclic propylphosphonate
N-benzoyl-8-bromo-2'-O-butyrylguanosine-3',5'-cyclic propylphosphonate
Explanation will be given hereinbelow with regard to pharmacological activities of the compounds according to the invention.

The inhibitory activity of cyclic AMP dependent phosphodiesterase was examined according to the method of W. Joseph et al. ("Method in Enzymology," 38, 205), by using bovine heart cyclic AMP phosphodiesterase (manufactured by Boehringer Mannheim.)

To a 40 mM Tris-hydrochloride buffer (pH=8) containing magnesium chloride at a final concentration of 5 mM and 2-mercaptoethanol at a final concentration of 3.75 mM were added 200,000 cpm of [³H]-labeled cyclic AMP, 0.125 to 100 µM of cyclic AMP, 1 µg of cyclic AMP dependent phosphodiesterase and a test compound at various concentrations (the total volume of the reaction mixture was 400 µl). After the reaction was allowed to proceed at 30 °C for 10 minutes, the reaction mixture was treated at 100 °C for 2.5 minutes so as to deactivate the enzyme. After the temperature of the reaction mixture had dropped to room temperature, 50 µg of snake venom (manufactured by Sigma Corp.) was added thereto, and the mixture was heated at 30 °C for 10 minutes to decompose 5′-AMP, which was formed by the action of cyclic AMP phosphodiesterase, to adenosine. To this was added 0.5 ml of a 1:3 (resin:H₂O) aqueous suspension of AG1-X2 resin (manufactured by Bio-Rad Co.) to terminate the decomposition, and the reaction mixture was allowed to stand at 4 °C for 15 minutes. Thereafter, the resulting mixture was centrifuged at 12,000 rpm for 2 minutes. The radioactivity of 500 µl of the supernatant was measured with a liquid scintillation counter, and the inhibition activity of the test compound was calculated. In the case of Compound 1, prepared in Example 1 described bereinbelow, marked inhibition activity was exhibited at a concentration of 100 µM.

Inhibitory action of the optically pure compounds of the present invention against cyclic GMP phosphodiesterase was tested by the similar manner too. It will be given as hereunder in detail.

Thus, the inhibitory action was tested in accordance with Pichard et al (J. Bio. Chem. vol.251, page 5726, 1976) using bovine heart cyclic nucleotide phosphodiesterase (manufactured by Boehringer/Mannheim). To be more precise, 50 µl of test compound to a final 10 µM concentration and 0.1 µg of cyclic nucleotide phosphodiesterase are added to 100 µl of 40mM Tris-hydrochloric acid buffer (pH 8.0) prepared so as to make the final concentrations of MgCl₂, 2-mercaptoethanol and ³H-cyclic GMP 5 mM, 0.25% and 1.82 x 10⁴ Bq (corresponding to 0.1 µl), respectively. The reaction is carried out by keeping at 30°C for 10 minutes, the enzyme is inactivated by heating at 97°C for 5 minutes, 50 µg of snake venom (Sigma) is added to the resulting 5′-GMP at room temperature, the mixture is kept at 30°C for 10 minutes to decompose into guanosine, then well mixed with 50 µl of a 1:3 aqueous suspension of AG 1 x 8 resin (Bio-Rad) and centrifuged at 14,000 rpm for 2 minutes.

The supernatant liquid (250 µl) is subjected to the radioactivity counting by liquid scintillation counter.

The enzymatic activity decreased as a result of the addition of the present invention compound was given in percentage taking the activity where no compound was added as a standard. Said percentage is defined as an "inhibitory activity" and the result is given in Table 1.

**Table 1**

| Compound Used | Inhibitory Activity(%) |
|---|---|
| [VIII] | 46 |
| [IX] | 32 |
| [X] | 61 |
| [XI] | 31 |

Stereochemical structures of the optically pure compounds used here together with some more which were synthesized in the examples given later are as follows:

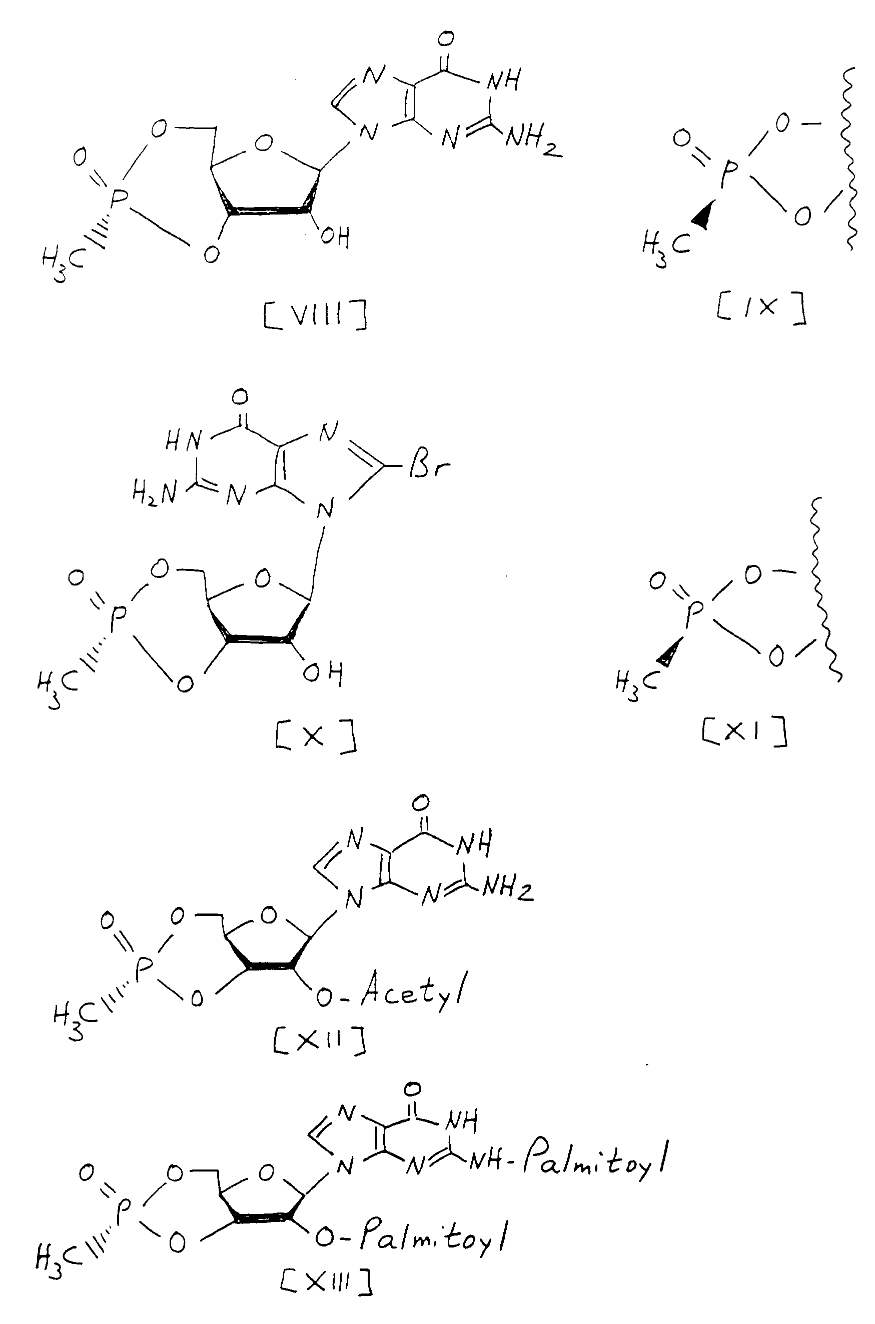

The compounds of the invention can be administered to animals, including human beings, as they are or in the form of a pharmaceutical composition comprising one or more pharmaceutically acceptable nontoxic inactive carriers and one or more of the compounds at a percentage of 0.1 to 99.5%, preferably 0.5 to 90%.

As carriers, there can be used one or more solid, semisolid or liquid diluents, fillers and/or other formulating auxiliaries. Pharmaceutical compositions according to the invention are administered preferably in unit dosage forms. They can be administered, e.g., orally, topically (e.g., transdermal delivery), rectally, or by intramuscular injection. It is a matter of course that the compounds of the invention should be administered in the form of a preparation suited for the particular mode of administration. Intramuscular administration can be particularly preferable.

Their optimum dosage as an antidementia agent may vary depending, e.g., on the age, weight, etc. of the patient, as well as on the mode of administration, nature and the state of the illness. Generally, the amount of active ingredients according to the invention to be administered to an adult is from 300 mg to 3 g/day/person, preferably from 500 mg to 1 g/day/person. There are of course cases where a smaller amount will suffice, or cases where a greater amount is required. It can be preferable to administer in 1 to 3 doses per day.

The cyclic nucleotide derivatives according to the invention can be synthesized, e.g., in accordance with the following reactions. 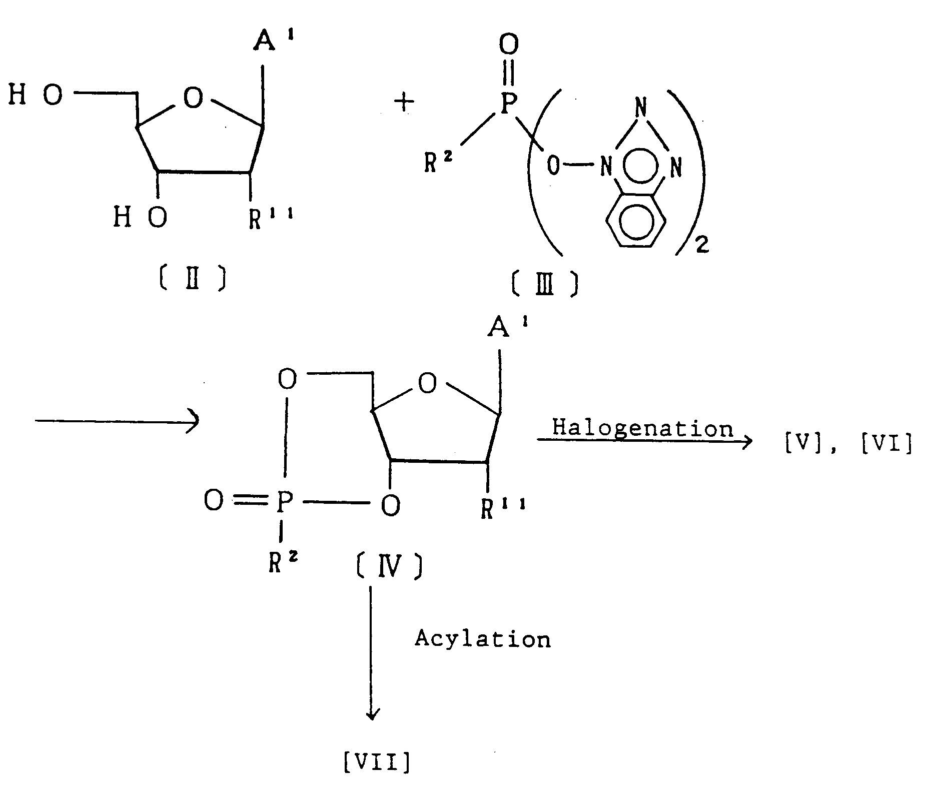
(in which R² has the same meanings as defined hereinabove; A¹ represents an adenine or guanine group; and R¹¹ represents a hydrogen atom or a tetrahydropyranyloxy group).

To be more specific, a nucleoside represented by General Formula [II] is allowed to react with an alkyl-O,O-bis-(1-benzotriazolyl)phosphonate represented by General Formula [III], followed by the addition of a 1-alkylimidazole, e.g., 1-methylimidazole, to give a cyclic derivative represented by General Formula [IV]. If necessary, the tetrahydropyranyl group contained in [IV] can be removed through reaction with an acid.

Thereafter, the acid-treated compound obtained from General Formula [IV] may be further subjected to halogenation or acylation to obtain a compound represented by General Formula [V] (8-halogenoadenosine derivative), General Formula [VI] (8-halogenoguanosine derivative) or General Formula [VII] (2′-acyloxy derivative).

The starting material [II] to be used in the above process can be prepared by the method described by S. Honda et al., Tetrahedron, 40, 153-163 (1984), or by a similar method. The compound represented by General Formula [III] can be obtained from an alkylphosphoric acid dichloride and 1-hydroxybenzotriazole in accordance with the method of J.H. van Boom et al., Nucleic Acid Research, 14, 2171-2185 (1986).

The reaction between Compound [II] and Compound [III] can be effected in an inactive solvent (e.g., aprotic solvent such as anhydrous dioxane, tetrahydrofuran, etc.), usually by allowing them to stand at room temperature for 30 minutes to 3 hours. Then, a 1-alkylimidazole compound is added thereto, and the resulting mixture is allowed to stand at room temperature for 5 to 24 hours.

The amount of [III] to be used is preferably from 1 to 1.2 times, based on mole, that of [II]. The amount of 1-alkylimidazoles to be used is 3 to 7 times, based on mole, that of the product formed by the reaction between Compounds [II] and [III].

Thereafter, a strong acid having a pH of ca. 2.0, preferably such a volatile acid as trifluoroacetic acid, hydrochloric acid, or the like, is added to the reaction mixture, and the resulting mixture is allowed to stand at a temperature of from 0 °C to room temperature for 5 to 24 hours to give the deprotected compound from [IV]. In this reaction, the acid is used usually in large excess against Compound [IV].

The deprotected compound from [IV] may be further subjected to halogenation In the case of bromination, for example, a compound from [IV] is treated with bromine water in an acetate buffer or with bromine water alone at a temperature of 0 °C to room temperature to give Compound [V] or [VI].

The acid-treated compound from General Formula [IV] can be converted into [VII] by acylating the former with an acid halide or an acid anhydride containing an acyl group corresponding to R³ or R⁵. This acylation can be conducted in pyridine at a temperature of from 0 °C to room temperature for a period of from a few hours to 24 hours.

The thus obtained desired compounds can be isolated and purified by known techniques, for example, by means of solvent extraction, adjustment of acidity or basicity of the solvent, solvent exchange, condensation, crystallization, recrystallization, chromatography, and the like.

### [Examples]

The present invention will be further illustrated by examples relating to the production of compounds according to the invention.

The following starting materials used in the examples were prepared as follows.

### N-monomethoxytrityl-2′-O-tetrahydropyranyladenosine:

This compound was prepared according to the method described by S. Honda et al., Tetrahedron, 40, 153-163 (1984). N-benzoyl-2′-O-tetrahydropyranyladenosine:

This compound was prepared from commercially available N-benzoyladenosine according to the method of S. Honda et al. described above.

### N-butyryl-2′-O-tetrahydropyranyladenosine:

This compound was prepared in the following manner. A solution of N-benzoyl-3′,5′-tetraisopropyldisiloxan-1,3-diyl-2′-O-tetrahydropyranyladenosine in methanol was treated overnight at room temperature with concentrated aqueous ammonia to give 3′,5′-tetraisopropyldisiloxan-1,3-diyl-2′-O-tetrahydopyranyladenosine, which was then treated overnight at room temperature with butyryl chloride in pyridine to give N-butyryl-3′,5′-O-tetraisopropyldisiloxan-1,3-diyl-2′-O-tetrahydropyranyladenosine. After the butyryl derivative had been dissolved in 13 ml of tetrahydrofuran, 13 ml of a tetrahydrofuran solution of 1 mol of tetrabutyl ammonium fluoride was added thereto, and the mixture was stirred at room temperature for 15 minutes. After the reaction, 100 ml of a mixture of dichloromethane and pyridine (3:1) was added thereto, and the resulting mixture was washed with a saturated aqueous sodium bicarbonate solution. The organic layer was dried with magnesium sulfate and concentrated to dryness. The residue was purified by silica gel chromatography (dichloromethane/methanol) to give the desired compound. N-monomethoxytrityl-2′-O-tetrahydropyranylguanosine was prepared according to the method of S. Honda et al. described above.

### Example 1: Synthesis of 8-bromoadenosine-3′,5′-cyclic methyl phosphonate

To 915 mg of N-monomethoxytrityl-2′-O-tetrahydropyranyladenosine was added at room temperature 20.5 ml of a 0.11 M dioxane solution of methyl-O,O-bis-(1-benzotriazolyl) phosphonate prepared by the method of J.H. van Boom et al., as described in Nucleic Acid Research, 14, 2171-2185 (1986). After 30 minutes, 1.4 ml of 1-methylimidazole was added thereto, and the reaction was allowed to proceed overnight. Thereafter, 2.0 ml of pyridine was added thereto, and the resulting mixture was distributed between dichloromethane and water (containing 1/10 volume of saturated aqueous sodium chloride solution and 1/10 volume of 1 M triethyl ammonium acetate [pH=7.0]), and the organic layer was dried with magnesium sulfate and concentrated to dryness to give crude solids. The product was purified by preparative TLC to give 310 mg of white crystals.

The product (310 mg) was dissolved in 20 ml of a mixture of dioxane and water (9:1), and 0.1 N hydrochloric acid was added dropwise thereto up to a pH of 2.0. This was allowed to stand overnight at room temperature. After the completion of the reaction had been confirmed by TLC, the reaction mixture was concentrated to dryness. The residue was purified by preparative TLC to give 125 mg of white solids. The product was recrystallized from a mixture of water and ethanol (5:95) to give white needles of adenosine-3′,5′-cyclic methyl phosphonate. Melting point: 207-210 °C.

In 0.4 ml of 0.5 M acetate buffer (pH = 4.0) was dissolved 10 mg of the compound prepared above, and 0.6 ml of bromine-water was added thereto at room temperature. After being allowed to stand for 1 hour, the reaction mixture was condensed to dryness, and the residue was purified by preparative TLC to give 11 mg of the desired compound.
TLC (methanol/dichloromethane [1:10]): Rf = 0.65
¹H-NMR (D₂O) δ : 1.92 (3H, d, J=18.0 Hz P-CH₃), 4.35 (1H, m, H-5a), 5.06 (1H, d, J=6.0, H-2′), 5.47 (1H, m, H-3′), 6.08 (1H, s, H-1′), and 8.12 (1H, s, Arom.H-2) 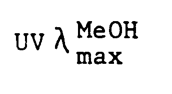
= 273 nm

### Example 2: Synthesis of N-benzoyladenosine-3′,5′-cyclic methyl phosphonate

To 93 mg of N-benzoyl-2′-O-tetrahydropyranyladenosine was added 2.7 ml of a 0.11 M solution in dioxane of methyl-O,O-bis-(1-benzotriazolyl) phosphonate prepared in a similar manner as in Example 1. After the resulting mixture had been allowed to stand at room temperature for 30 minutes, 0.2 ml of 1-methylimidazole was added thereto, and the reaction was allowed to proceed overnight at room temperature. Thereafter, 1.0 ml of pyridine was added, and the resulting mixture was treated in the same manner as in Example 1 to give 40 mg of white solids of N-benzoyl-2′,O-tetrahydropyranyladenosine-3′,5′-cyclic methylphosphonate.

In 4.0 ml of a mixture of dioxane and water (9:1) was dissolved the above product (40 mg), and 1.4 ml of 0.1N hydrochloric acid was added dropwise at room temperature. After the reaction mixture had been allowed to stand overnight to complete the reaction, the reaction mixture was condensed to dryness, and the residue was purified by means of preparative TLC to give 32 mg of white solids.
¹H-NMR (CDCl₃/CD₃OD [8:2]) δ : 1.76 (3H, d, J=18.0 P-CH₃), 4.30-4.7 (4H, m), 4.84 (1H, d, J=5.0, H-2′), 5.30 (1H, m, H-3′), 6.11 (1H, s, H-1′), 7.50-7.70 (3H, benzoyl), 8.00-8.10 (2H, benzoyl), 8.34 (1H, s, Arom.), and 8.76 (1H, s, Arom.)

### Example 3: Synthesis of N-butyryladenosine-3′,5′-cyclic methyl phosphonate

White solids of N-butyryl-2′-O-tetrahydropyranyladenosine-3′,5′-cyclic methylphosphonate (45 mg) was prepared from 134 mg of N-butyryl-2′-O-tetrahydropyranyladenosine in a similar manner as in Example 1.

The product was subjected to acid hydrolysis in a similar manner as in Example 1 to give 30 mg of the desired compound.
¹H-NMR (D₂O) δ: 1.00 (3H, t, J=7.8 CH₂CH₃), 1.80 (2H, hextet, J=7.8 CH₂CH₃), 1.86 (3H, d, J=18.0 P-CH₃), 2.60 (2H, t, J=7.8,-CO-CH₂), 4.92 (1H, d, J=4.0, H-2′ 5.20 (1H, m, H-3′), 6.25 (1H, s, H-1′), 8.46 (1H, s, Arom), and 8.65 (1H, s, Arom)

### Example 4: Synthesis of N-benzoyl-2′-O-deoxyadenosine-3′,5′-cyclic methylphosphonate

To 355 mg of commercially available N-benzoyl-2′-deoxyadenosine was added at room temperature 14 ml of a 0.11 M solution in dioxane of methyl-O,O-bis(1-benzotriazole) phosphonate prepared in a similar manner as in Example 1. After 30 minutes, 1.0 ml of 1-methylimidazole was added thereto. After the mixture had been further stirred overnight at room temperature, 2.0 ml of pyridine was added thereto, and the resulting mixture was distributed between 100 ml of a mixture of dichloromethane and dioxane (9:1) and a saturated aqueous sodium chloride solution. The organic layer was condensed to dryness, and the residue was purified by silica gel chromatography to give 114 mg of white solids of the desired compound.

### Example 5: Synthesis of guanosine-3′,5′-cyclic methylphosphonate

To 280 mg of N-monomethoxytrityl-2′-O-tetrahydropyranylguanosine was added at room temperature 8 ml of a 0.11 M solution in dioxane of methyl-O,O-bis-(1-benzotriazole) phosphonate prepared in a similar manner as in Example 1. After 30 minutes, 0.6 ml of 1-methylimidazole was added thereto. After the reaction had been allowed to proceed overnight at room temperature, 1.0 ml of pyridine was added, and the resulting mixture was distributed between dichloromethane and a saturated sodium chloride solution. The organic layer was condensed to dryness, and the residue was purified by means of preparative TLC to give 60 mg of white solids of N-monomethoxytrityl-2′-O-tetrahydropyranylguanosine-3′5′-cyclic methylphosphonate.

In 4.0 ml of a mixture of dioxane and water (9:1) was dissolved 40 mg of the compound, and the pH of the solution was adjusted to 2.0 with 0.1 N hydrochloric acid. After being allowed to stand overnight at room temperature, the reaction mixture was condensed to dryness and purified by TLC to give 19 mg of the desired compound.
Melting point: 238-240 °C (decomp.)
¹H-NMR (D₂O) δ: 1.82 (3H, d, J=18.0 P-CH₃), 4.34-4.90 (4H, m) 5.20 (1H, m, H-3′), 5.98 (1H, s, H-1′), and 7.85 (1H, s, H-8)
UVλₘₐₓ: 252 nm (ε = 13,200)

### Example 6: Synthesis of 8-bromoguanosine-3′,5′-cyclic methyl phosphonate

In 0.5 ml of water was added 2.0 mg of guanosine-3′,5′-cyclic methyl phosphonate prepared in Example 5. To this was added 0.2 ml of bromine-water, and the reaction was allowed to proceed at room temperature for 5 minutes. It was confirmed by means of TLC that the starting material had completely disappeared [TLC: methanol/dichloromethane (2:8), Rf value of the starting material = 0.18, and Rf value of the product = 0.45]. The mixture was condensed to dryness, and the residue was purified by TLC to give 2.5 mg of the desired compound. 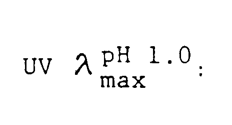 261 nm (ε = 18,500)

### Example 7: Synthesis of N-n-butyryl-2′-O-n-butyryladenosine 3′,5′-cyclic methylphosphonate

At room temperature, 60 µl of n-butyryl chloride was added to 0.6 ml of a pyridine solution of 5 mg of adenosine-3′,5′-cyclic methylphosphonate. After being allowed to stand overnight, the mixture was condensed to dryness, and the residue was distributed between dichloromethane and water. The organic layer was dried and condensed to dryness, and the residue was purified by preparative TLC dichloromethane : methanol = 95:5 to give 3 mg of the desired compound.
¹H-NMR (CDCl₃) δ: 1.72 (3H, d, J=19.0 p-CH₃), 2.60 (2H, t, NHCO-CH₂-), 2.84 (2H, t, OCO-CH₂-), 5.28 (1H, d, H-2′), 6.02 (1H, s, H-1′), 8.10 (1H, s, Arom), and 8.71 (1H, s, Arom)

### Example 8: Synthesis of [VIII] and [IX]

Guanosine-3′,5′-cyclic methylphosphonate (90 mg) prepared in Example 5 was dissolved in 20 ml of water, the solution was applied to silicagel (40 ml; 20-80 µm) for reversed phase chromatogrphy, and eluted with water, 5% aqueous methanol, 10% aqueous methanol and 15% aqueous methanol successively whereupon 8 mg of [IX] was eluted firstly and then 75 mg of [VIII] was eluted. Thus they were eluted in completely separated manner.
[VIII]: 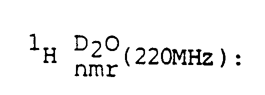
1.82 (3H, d, J = 19.0, p-CH₃), 4.34-4.90 (4H, m), 5.20 (1H, m, H-3′), 5.80 (1H, s, H-1′), 7.86 (1H, s, H-8).
[IX]: 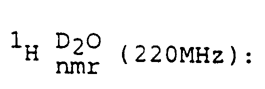
1.88 (3H, d, J = 18.0, p-CH₃), 4.34-4.90 (5H, m), 6.02 (1H, s, H-1′), 7.86 (1H, s, H-8).

### Example 9: Synthesis of [X] and [XI]

[VIII] (32 mg) prepared in Example 8 was made to react with 1.0 ml bromine water at room temperature for 5 minutes in 1.0 ml of water. Bromine was evaporated using an aspirator, the resulting precipitate was collected, washed with cold water and rinsed with acetone to give 30 mg of [X], yellow powder.

Similarly prepared was 28 mg [XI], yellow powder, from 32 mg of [IX].

### Example 10: Synthesis of [XII]

[VIII] (30 mg) prepared in Example 8 was allowed to stand overnight with a mixture of 0.5 ml of acetic anhydride and 1.0 ml of pyridine at room temperature, the reaction mixture was concentrated in vacuo followed by evaporating to dryness and the residue was pulverized with a 100:5:0.5 mixture of n-hexane, dichloromethane and methanol to give 32 mg of [XII]. 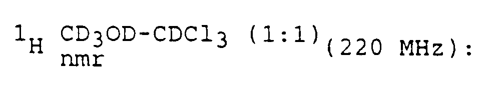
1.75 (3H, d, J = 18.0, p-CH₃), 2.20 (3H, s, OCOCH₃), 4.00-4.65 (3H, m), 5.80 (1H, d, J = 8.0, H-1′), 5.75-5.85 (2H, m, H-2′,3′ overlapped with H-1′), 7.60 (1H, bs, H-8).

### Examle 11: Synthesis of [XIII]

[VIII] (30 mg) prepared in Example 8 was suspended in 3.0 ml of pyridine and made to react with 72 mg of palmitoyl chloride for 12 hours. After the reaction, the mixture was concentrated and partitioned between dichloromethane and diluted hydrochloric acid. The organic layer was concentrated and evaporated to dryness. The residue was purified with preparative TLC (20 x 20 cm; 2mm thickness)using 5% methanol-methylene dichloride as a developing solvent to give 40 mg of [XIII]. --

## Claims

1. Compounds represented by General Formula [I]: 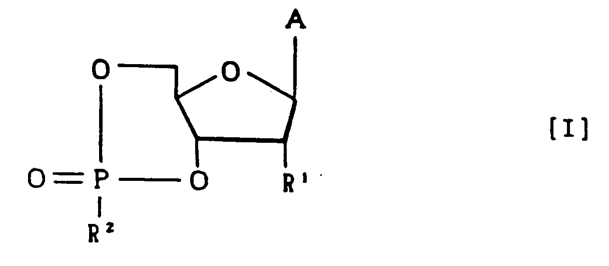 wherein R¹ represents a hydrogen atom, a hydroxyl group or an acyloxy group; R² represents an alkyl group; and A represents: 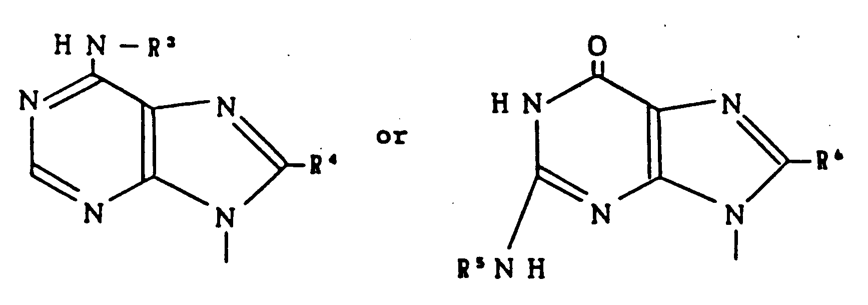 (in which R³ represents a hydrogen atom or an acyl group; R⁴ represents a hydrogen atom or a halogen atom; R⁵ represents a hydrogen atom or an acyl group; and R⁶ represents a hydrogen atom or a halogen atom), excluding the one in which R¹ is hydroxyl, R² is alkyl, and A is: 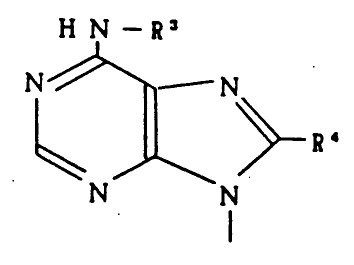 (in which R³ is hydrogen and R⁴ is hydrogen).

2. The compound of claim 1 in which said compound is optically pure.

## Patentansprüche

1. Verbindungen der allgemeinen Formel [I]: 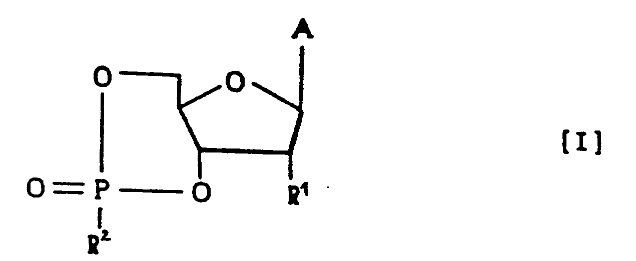 wobei R¹ ein Wasserstoffatom, eine Hydroxyl-Gruppe oder eine Acyloxy-Gruppe ist; R² ist eine Alkyl-Gruppe; und A ist 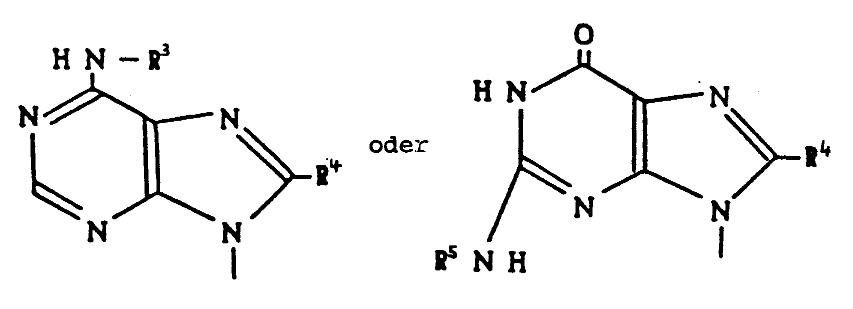 (wobei R³ ein Wasserstoffatom oder eine Azyl-Gruppe ist; R⁴ ist ein Wasserstoffatom oder ein Halogenatom; R⁵ ist ein Wasserstoffatom oder eine Azyl-Gruppe; und R⁶ ist ein Wasserstoffatom oder ein Halogenatom),
außer dem Fall, bei dem R¹ ein Hydroxyl ist, R² ist ein Alkyl und A ist: 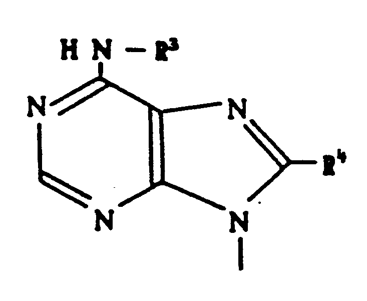 (wobei R³ Wasserstoff ist und R⁴ ebenfalls Wasserstoff ist).

2. Verbindung nach Anspruch 1,
wobei die Verbindung optisch rein ist.

## Revendications

1. Les composés de formule générale I: 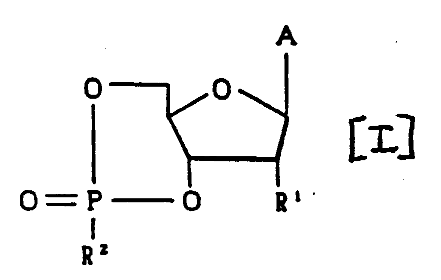 dans laquelle R¹ représent un atome d'hydrogène, un groupement hydroxy ou acyloxy; R² représent un radical alkyle; et A représent: 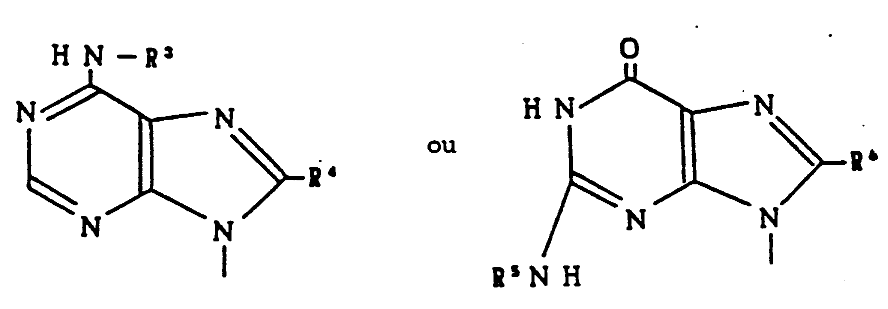 (dans laquelle R³ représent un atome d'hydrogène ou un radical acyle; R⁴ représent un atome d'hydrogène ou un atome d'halogène; R⁵ représent un atome d'hydrogène ou un radical acyle; et R⁶ représent un atome d'hydrogène ou un atome d'halogène) à l'exception de celui dans laquelle R¹ est hydroxy, R² est alkyle et A est: 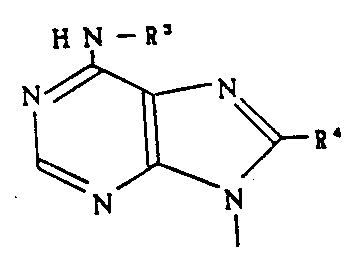 (dans laquelle R³ est hydrogène et R⁴ est hydrogène).

2. Le composé selon la revendication 1, quel composé est de pureté optique.
